(19) Europäisches Patentamt
European Patent Office
Office européen des brevets

(11) Numéro de publication : **0 457 022 B1**

(12) **FASCICULE DE BREVET EUROPEEN**

(45) Date de publication du fascicule du brevet : **03.08.94 Bulletin 94/31**

(51) Int. Cl.[5] : **C07C 31/135,** C07C 29/143, C07C 29/00, A61K 7/46

(21) Numéro de dépôt : **91105652.1**

(22) Date de dépôt : **10.04.91**

# (54) Alcools aliphatiques optiquement actifs nouveaux et leur utilisation à titre d'ingrédients parfumants.

(30) Priorité : **16.05.90 CH 1671/90**

(43) Date de publication de la demande : **21.11.91 Bulletin 91/47**

(45) Mention de la délivrance du brevet : **03.08.94 Bulletin 94/31**

(84) Etats contractants désignés : **CH DE FR GB LI NL**

(56) Documents cités :
**EP-A- 0 118 809**
**EP-A- 0 121 828**
**HELVETICA CHIMICA ACTA. vol. 68, no. 7, 1985, BASEL CH pages 1961 - 1985; K.H. Schulte-Elte et al.: "Diastereoselektivität der Geruchswahrnehmung von Alkoholen der Iononreihe"**
**DRAGOCO REPORT. vol. 27, no. 8-9, 1980, HOLZMINDEN DE pages 199 - 203; E-J. Brunke et al.: "Über chemische Struktur und Geruchseffekt in der Tetrahydroionol-Reihe"**
**Theilheimer's Synthetic Methods of Organic Chemistry vol. 15, 1961, S. Karger, Bâle CH**
**Theilheimer's Synthetic Methods of Organic Chemistry vol. 9, 1955, S. Karger, Bâle CH**

(73) Titulaire : **FIRMENICH SA**
**1, route des Jeunes**
**CH-1211 Genève 8 (CH)**

(72) Inventeur : **Schulte-Elte, Karl-Heinrich**
**44, chemin de Carabot**
**CH-1213 Onex (CH)**
Inventeur : **Margot, Christian**
**Chemin de la Ravière**
**CH-1261 Bassins (CH)**
Inventeur : **Chapuis, Christian**
**Vy de Montorge 5**
**CH-1295 Mies (CH)**
Inventeur : **Simmons, Dana P.**
**1105 Edinburgh Court**
**Jamestown, North Carolina 27282 (US)**
Inventeur : **Reichlin, Daniel**
**253A, rue de Bernex**
**CH-1232 Confignon (CH)**

(74) Mandataire : **Salvadori, Giuseppe, Dr.**
**c/o Firmenich S.A.**
**Case Postale 239**
**CH-1211 Genève 8 (CH)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a trait au domaine de la parfumerie. Elle concerne, plus particulièrement, des isomères optiquement actifs nouveaux de deux alcools aliphatiques représentés par la formule

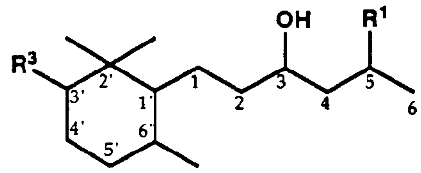

(I)

dans laquelle $R^3$ représente un atome d'hydrogène ou un radical méthyle et le symbole $R^1$ représente un atome d'hydrogène, à savoir, les 1-(2′,2′,6′-triméthyl-1′-cyclohexyl)-3-hexanol et 1-(2′,2′,3′,6′-tétraméthyl-1′-cyclohexyl)-3-hexanol.

Les alcools de formule (I) ci-dessus, ainsi que leurs stéréoisomères, sont des composés connus. Par exemple, le brevet EP 118 809, dont la présente demanderesse est titulaire, décrit les isomères de configuration cis et trans du 1-(2′,2′,6′-triméthyl-1′-cyclohexyl)-3-hexanol, ainsi que leurs propriétés odorantes. D'autre part, le brevet EP 121 828, appartenant également à la demanderesse, a pour objet le 1-(2′,2′,3′,6′-tétraméthyl-1′-cyclohexyl)-3-hexanol, ses isomères de configuration et leur utilisation en parfumerie. Il ressort de ces deux brevets que, d'une façon générale, les isomères de configuration trans, et notamment ceux possédant les trois substituants du cycle hexanique (positions 1′, 3′, et 6′) en position équatoriale, sont jugés nettement supérieurs à leurs homologues de configuration cis, du point de vue de leurs propriétés odorantes. Ces dernières sont d'ailleurs distinctes d'un isomère de configuration à l'autre.

L'art antérieur mentionné ne concernait que des stéréoisomères des alcools cités se présentant sous forme de mélanges racémiques. Or, les alcools de formule (I) présentent plusieurs centres chiraux et leurs racémates sont des mélanges des différents isomères optiquement actifs possibles. Rien n'était dit dans l'art antérieur sur un intérêt éventuel que ces isomères pourraient présenter du point de vue olfactif. L'un des objectifs de l'homme du métier dans l'industrie des parfums est de trouver des composés nouveaux ayant des qualités olfactives supérieures à celles des composés connus, soit de par le fait que leurs propriétés odorantes sont plus distinctives et originales, soit en raison de leur odeur beaucoup plus intense, soit encore de par ces deux facteurs. Il sait bien, cependant, qu'il ne peut pas se fier à des critères basés sur la similarité structurelle pour prévoir si un composé nouveau sera potentiellement plus intéressant, en tant qu'ingrédient parfumant ou si, en fait, il aura même une odeur quelconque.

Bien que cet homme du métier n'ignore pas le rôle que l'isomérie optique d'un composé joue dans son activité olfactive, ou mieux, dans sa perception olfactive chez le parfumeur [voir, par exemple, W. Pickenhagen dans ACS Symposium Series 388, chapitre 12, p. 151, ed. ACS, Washington DC (1989)], il sait tout aussi bien que, particulièrement lorsqu'il est confronté à des composés racémiques possédant plusieurs centres chiraux, sa tâche sera extrêmement ardue et non évidente car la synthèse des isomères optiquement actifs purs correspondants requiert des compétences spéciales et est très coûteuse, sans que l'on puisse prévoir à priori, même en présence d'un racémate de bonne qualité olfactive, si ses efforts mèneront uniquement à la découverte qu'aucun des composants optiquements actifs purs du racémate ne possède des propriétés odorantes jugées supérieures à celles de ce dernier ou, du moins, pas suffisamment supérieures pour justifier son développement industriel, nécessairement plus onéreux.

Malgré ces difficultés, la synthèse de nouveaux ingrédients parfumants chiraux est un but toujours plus poursuivi dans ce domaine. La raison de ceci ressort clairement de publications telles que celle de G. Ohloff dans Experientia 42, 271 (1986), lequel fait remarquer au lecteur l'importance de la découverte de tout nouveau composé odorant, pour une définition plus complète des listes empiriquement établies de conditions ou paramètres structurels, dont l'activité chirale, lesquels, croit-on savoir, jouent un rôle dans la perception des odeurs. Cependant, cet auteur reconnaît également le caractère transitoire, dans l'état présent de la technique, de ces listes de paramètres qui peuvent être facilement contredites ou bouleversées par les propriétés inattendues de ces mêmes composés nouvellement découverts (voir par exemple, la conclusion à la page 277).

La présente invention apporte justement une contribution nouvelle et surprenante dans ce domaine. Ainsi, c'est de façon surprenante que nous venons de découvrir que des propriétés odorantes, notamment la puissance olfactive, associées jusqu'ici aux isomères de configuration trans des alcools (I) précités, sous leur forme racémique, ne sont en fait pas inhérents à tous les composants optiquement actifs de ces racémates. Bien au contraire, chacun des isomères optiquement actifs nouveaux, faisant l'objet de la présente invention, possède une odeur propre et distincte de celle des autres et dont l'intensité, surtout, peut varier dans une échelle

très étendue. Par ailleurs, il est de ces espèces optiquement actives qui présentent de nets avantages, pour l'utilisation en parfumerie, sur les mélanges racémiques correspondants, connus de l'art antérieur.

Ainsi, la présente invention a pour objet un composé de formule (I),

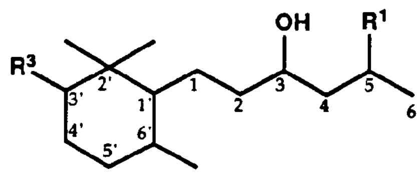

(I)

dans laquelle le symbole $R^3$ représente un atome d'hydrogène et le symbole $R^1$ représente un atome d'hydrogène ou un radical méthyle, sous forme d'un mélange optiquement actif d'épimères en C(3) de formule

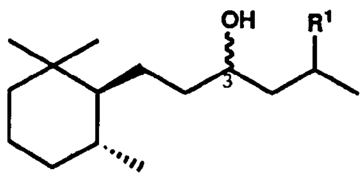

ou

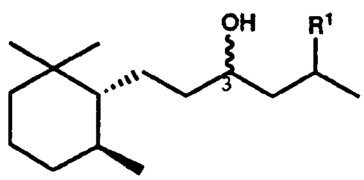

(-)-(1'S,6'R)
(Ia)

(+)-(1'R,6'S)
(Ib)

dans laquelle la ligne ondulée indique l'une ou l'autre des deux orientations possibles du groupe OH, ou sous forme de l'un des épimères en C(3) composant ledit mélange, ou dans laquelle le symbole $R^3$ représente un radical méthyle et le symbole $R^1$ représente un atome d'hydrogène, sous forme d'un mélange optiquement actif d'épimères en C(3) de formule

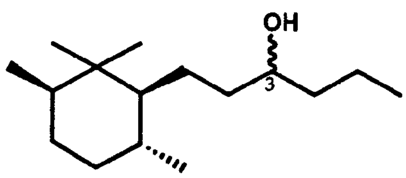

ou

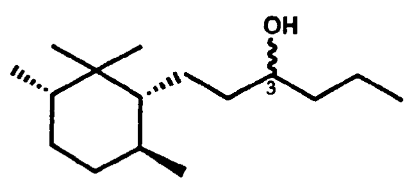

(-)-(1'S,3'R,6'R)
(Ic)

(+)-(1'R,3'S,6'S)
(Id)

dans laquelle la ligne ondulée a le sens indiqué ci-dessus, ou de l'un des épimères en C(3) composant ledit mélange.

Les composés de formules (Ia) à (Id) possèdent tous des notes odorantes de type boisé mais dont le caractère et la puissance varient d'un composé à l'autre. Par exemple, le mélange d'épimères de formule (Id), ou (+)-(1'R,3'S,6'S)-1-(2',2',3',6'-tétraméthyl-1'-cyclohexyl)-3-hexanol, un composé préféré de l'invention, possède une note odorante boisée-ambrée d'une extraordinaire puissance, accompagnée d'une légère sous-note de transpiration. De par ses propriétés odorantes, ce composé remplace avantageusement le 1-(2',2',c-3',t-6'-tétraméthyl-r-1'-cyclohexyl)-3-hexanol décrit dans EP 121 828, comme il ressort de l'exemple de comparaison correspondant présenté plus loin. Pour sa part, le mélange d'épimères de formule (Ic), ou (-)-(1'S,3'R,6'R)-1-(2',2',3',6'-tétraméthyl-1'-cyclohexyl)-3-hexanol, exhale une odeur de caractère similaire mais beaucoup plus faible.

Quant aux mélanges d'épimères de formules (Ia) et (Ib) dans lesquelles le symbole $R^1$ représente un atome d'hydrogène, ils possèdent des notes odorantes boisées plus élégantes et moins animales que celles de leurs homologues méthylés, en position 3' du cycle, cités ci-dessus. Il s'agit également de notes moins sèches et beaucoup moins puissantes que celles de ces derniers. Par ailleurs, nous avons aussi constaté une grande variété de puissances olfactives parmi les quatre isomères optiquement actifs de formule

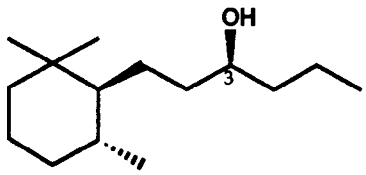

ou

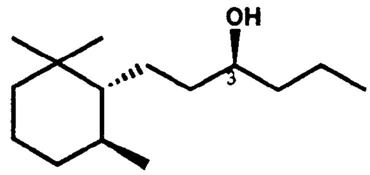

(-)-(1'S,3S,6'R)
(IIa)

(+)-(1'R,3S,6'S)
(IIb)

ou

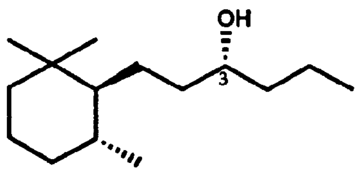

ou

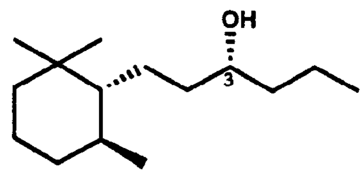

(-)-(1'S,3R,6'R)
(IIc)

(+)-(1'R,3R,6'S)
(IId)

présentant tous une configuration trans. C'est ainsi que le (+)-(1'R,3S,6'S)-1-(2',2',6'-triméthyl-1'-cyclohexyl)-3-hexanol se révèle être le meilleur composé de la série, possédant une note beaucoup plus puissante et tenace que celle des autres isomères. Il s'agit, en effet, d'une note boisée-ambrée très élégante, qui remplace avantageusement celle du trans-1-(2',2',6'-triméthyl-1'-cyclohexyl)-3-hexanol décrit dans EP 118 809, car elle est plus puissante et tenace que cette dernière, tout en ayant un caractère encore plus boisé.

Le composé de formule (IIa), ou (-)-(1'S,3S,6'R)-1-(2',2',6'-triméthyl-1'-cyclohexyl)-3-hexanol possède une note odorante de caractère semblable à celle du composé (IIb) cité plus haut, mais dont la puissance est déjà nettement inférieure. Quant aux épimères C(3) de ces deux composés, de formules (IIc) et (IId), leurs notes odorantes sont pratiquement dénuées de caractère animal et leur puissance olfactive est étonnamment faible et très inférieure à celle du composé (IIb) cité à titre préférentiel.

D'autre part, les mélanges d'épimères en C(3) de formule (Ia) ou (Ib), dans laquelle le symbole $R^1$ représente un radical méthyle, présentent également des notes de type boisé ressemblant à celles de leurs homologues non méthylés en position 5 de la chaîne. La puissance de la note du composé de formule (Ib) telle que définie ci-dessus, ou (+)-(1'R,6'S)-5-méthyl-1-(2',2',6'-triméthyl-1'-cyclohexyl)-3-hexanol, est très supérieure à celle du (-)-(1'S,6'R)-5-méthyl-1-(2',2',6'-triméthyl-1'-cyclohexyl)-3-hexanol de formule (Ia) définie ci-dessus.

Lorsqu'on compare les propriétés odorantes des deux composés préférés de l'invention, à savoir les (+)-(1'R,3'S,6'S)-1-(2',2',3',6'-tétraméthyl-1'-cyclohexyl)-3-hexanol et (+)-(1'R,3S,6'S)-1-(2',2',6'-triméthyl-1'-cyclohexyl)-3-hexanol, on constate que leur utilisation dans la préparation de compositions parfumantes et articles parfumés est autant fonction de l'effet parfumistique recherché que du goût du parfumeur les utilisant. En effet, bien que le composé cité en deuxième lieu soit jugé d'un usage plus facile en raison de la moindre puissance de son odeur, et qu'il soit préféré lorsqu'on recherche un effet plus élégant, la note plus agressive et animale du (+)-(1'R,3'S,6'S)-1-(2',2',3',6'-tétraméthyl-1'-cyclohexyl)-3-hexanol est tout aussi appréciée des parfumeurs pour des compositions parfumantes de type plus masculin.

Les composés selon l'invention peuvent trouver un domaine d'application fort étendu, soit par addition directe aux produits que l'on veut parfumer soit, plus fréquemment, en mélange avec d'autres ingrédients parfumants, des solvants ou des adjuvants usuels en parfumerie.

Les concentrations dans lesquelles ils peuvent être employés dans la préparation de compositions parfumantes dépendent, bien entendu, de l'effet odorant désiré et de la nature des autres coingrédients parfumants, ainsi que de la puissance de la note odorante du composé de l'invention en question. A titre d'exemple, on peut citer des concentrations de 5 à 10%, voire même 20% en poids, par rapport au poids de la composition, mais les exemples d'application présentés plus loin montrent que ces concentrations peuvent être bien inférieures à ces valeurs, notamment dans le cas des (+)-(1'R,3S,6'S)-1-(2',2',6'-triméthyl-1'-cyclohexyl)-3-hexanol et (+)-(1'R,3'S,6'S)-1-(2',2',3',6'-tétraméthyl-1'-cyclohexyl)-3-hexanol cités à titre préférentiel. Ce dernier est utilisé dans des concentrations encore moindres que celles de son homologue non méthylé.

Les composés de l'invention conviennent également bien pour le parfumage d'articles divers tels les savons, gels de douche ou bain, shampoings ou après-shampoings, préparations cosmétiques, désodorisants, détergents ou revitalisants textiles ou les produits d'entretien.

Selon l'invention, on peut préparer un mélange optiquement actif d'épimères en C(3) de formules (Ia) à (Id) suivant un procédé original caractérisé en ce qu'on réduit, à l'aide d'un aluminohydrure de métal alcalin, une cétone sous forme de l'un des énantiomères optiquement actifs de formule

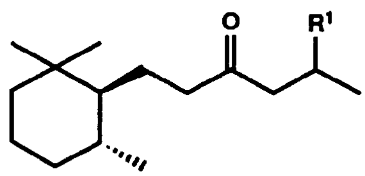

(-)-(1'S,6'R)
(IIIa)

ou

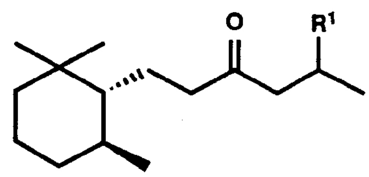

(+)-(1'R,6'S)
(IIIb)

dans laquelle le symbole $R^1$ représente un atome d'hydrogène ou un radical méthyle, ou, repectivement, de formule

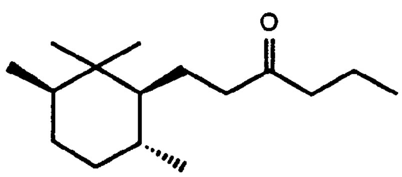

(-)-(1'S,3'R,6'R)
(IIIc)

ou

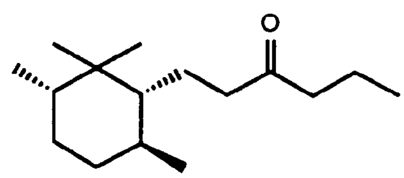

(+)-(1'R,3'S,6'S)
(IIId)

pour obtenir un mélange d'épimères de formule (Ia) ou (Ib) ou, respectivement, un mélange d'épimères de formule (Ic) ou (Id).

Les cétones de formules (IIIa) à (IIId), utilisées comme produits de départ dans le procédé de l'invention, peuvent être obtenues par condensation aldolique avec la 2-pentanone ou, le cas échéant, la 4-méthyl-2-pentanone, d'un aldéhyde, sous forme d'un isomère optiquement actif approprié, suivie d'une hydrogénation, comme il est illustré au schéma suivant :

Schéma I

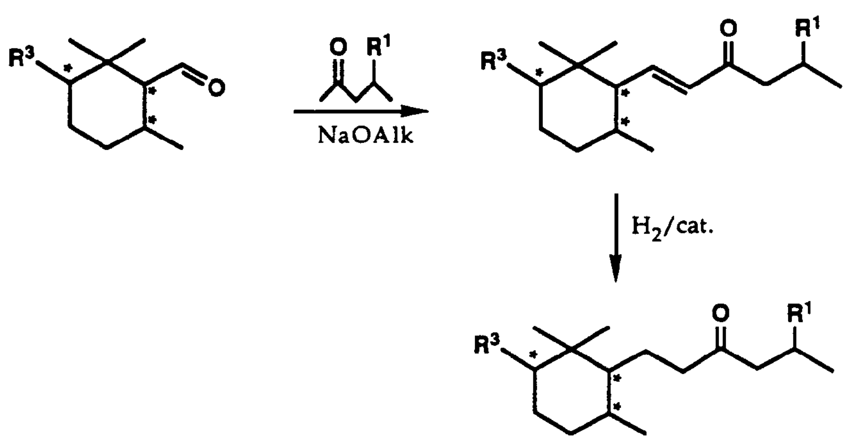


(III)

$R^1$ = H et $R^3$ = $CH_3$; ou
$R^1$ = H, $CH_3$ et $R^3$ = H

Alk = $CH_3$, $C_2H_5$
* = centre chiral

Les conditions spécifiques de ces réactions sont décrites en détail dans les exemples de préparation.

Les aldéhydes optiquement actifs de départ de formule

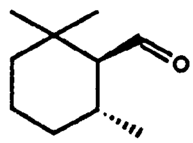

ou

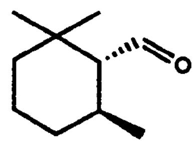

(-)-(1S,6R) (+)-(1R,6S)

peuvent être obtenus à partir de citronellal optiquement actif selon le procédé décrit dans le brevet européen No. 255 904.

D'autre part, les aldéhydes de formule

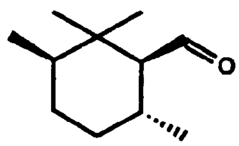

ou

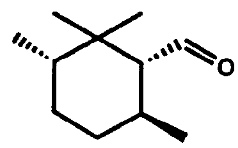

(-)-(1S,3R,6R) (+)-(1R,3S,6S)

peuvent être préparés selon la méthode décrite dans la demande de brevet européen publiée sous le n° 374509 (déposée par la demanderesse le 23.11.89 et portant la priorité suisse du 21.12.88), dont le contenu est inclu ici par référence. Les conditions spécifiques de préparation de ces aldéhydes sont décrites en détail dans les exemples présentés plus loin.

La réduction de la cétone de formules (IIIa) à (IIId) s'effectue de façon classique, à l'aide par exemple de $LiAlH_4$, et les conditions spécifiques de ces réactions sont également détaillées dans les exemples de préparation.

Nous avons, en outre, constaté que lorsqu'on désirait préparer un composé selon l'invention de formules (IIa) à (IId) ou l'un de ses homologues méthylés, il était préférable de suivre un autre procédé faisant également l'objet de la présente invention. Il s'agit alors d'un procédé caractérisé en ce qu'on fait réagir une hydroxy-sulfone de formule

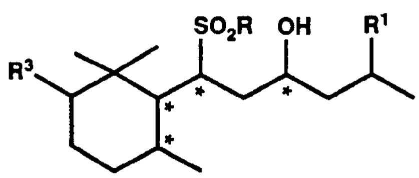

(IV)

dans laquelle l'astérisque indique la position d'un centre chiral, le symbole R représente un radical alkyle ou aryle et les symboles $R^1$ et $R^3$ sont définis comme à la formule (I), sous forme d'un isomère optiquement actif approprié, avec un agent de clivage réducteur du groupe sulfonyle et on sépare le composé désiré du produit de la réaction.

Il est connu de l'art antérieur que l'on peut obtenir le clivage d'une liaison C-S à l'aide d'un métal alcalin, notamment le lithium. Par exemple, C. G. Screttas et al., dans J. Org. Chem. 43, 1064 (1978) ont décrit des réactions de clivage d'alkyle phényle sulfures à l'aide d'une dispersion de lithium, en présence d'une quantité catalytique de naphtalène, dans le THF (tétrahydrofurane). D'autres conditions de réaction ont été divulguées par R. J. Armstrong et al., dans Can. J. Chem. 61, 2530 (1983) (et références y citées). Ces auteurs ont fait état de l'hydrogénolyse d'une hydroxy-sulfone à l'aide d'un amalgame à 6% de Na-Hg.

Selon un mode d'exécution préférentiel du procédé de la présente invention, on fait réagir un isomère optiquement actif approprié d'une hydroxy-sulfone de formule (IV), dans laquelle le symbole R représente un radical phényle, avec du naphtalure de lithium, dans le tétrahydrofurane. La réaction est régiospécifique et on obtient comme produit final l'isomère optiquement actif de formules (IIa) à (IId) ou l'un de ses homologues

méthylés, correspondant à l'isomère optiquement actif de l'hydroxy-sulfone (IV) de départ, accompagné d'une quantité mineure de son homologue de chaîne insaturée de formule

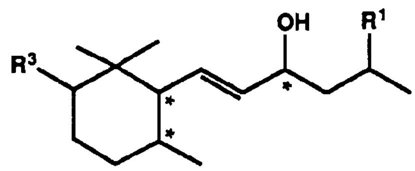

(V)

* = centre chiral
$R^1$, $R^3$ = définis à la formule (I)

Cet alcool allylique peut ensuite être séparé du mélange par acétylation de ce dernier, suivie d'ozonolyse, réduction et séparation chromatographique, comme il est décrit dans les exemples de préparation présentés plus loin. Ce traitement fournit le composé de formule (IIa), (IIb), (IIc) ou (IId), ou un homologue méthylé de celui-d, désiré à l'état pur.

D'autres conditions de réaction que celles du mode d'exécution préférentiel ont également été étudiées. On peut citer à cet effet, la réduction du groupe phénylsulfonyle à l'aide d'une suspension de lithium dans l'éthylamine, ou encore d'un amalgame à 6% de Na-Hg dans les conditions répertoriées dans l'art antérieur. Ces deux méthodes conviennent également à la préparation des composés de formules (IIa) à (IId) selon l'invention. Elles sont néanmoins moins rentables que le mode d'exécution préféré, en raison du fait qu'elles conduisent à des mélanges plus riches en produit secondaire (V).

Les hydroxy-sulfones optiquement actives de formule (IV), dont la pureté optique conditionne celle du produit final désiré, peuvent être obtenues selon le procédé illustré au schéma suivant pour un isomère optiquement actif déterminé :

<u>Schéma II</u>

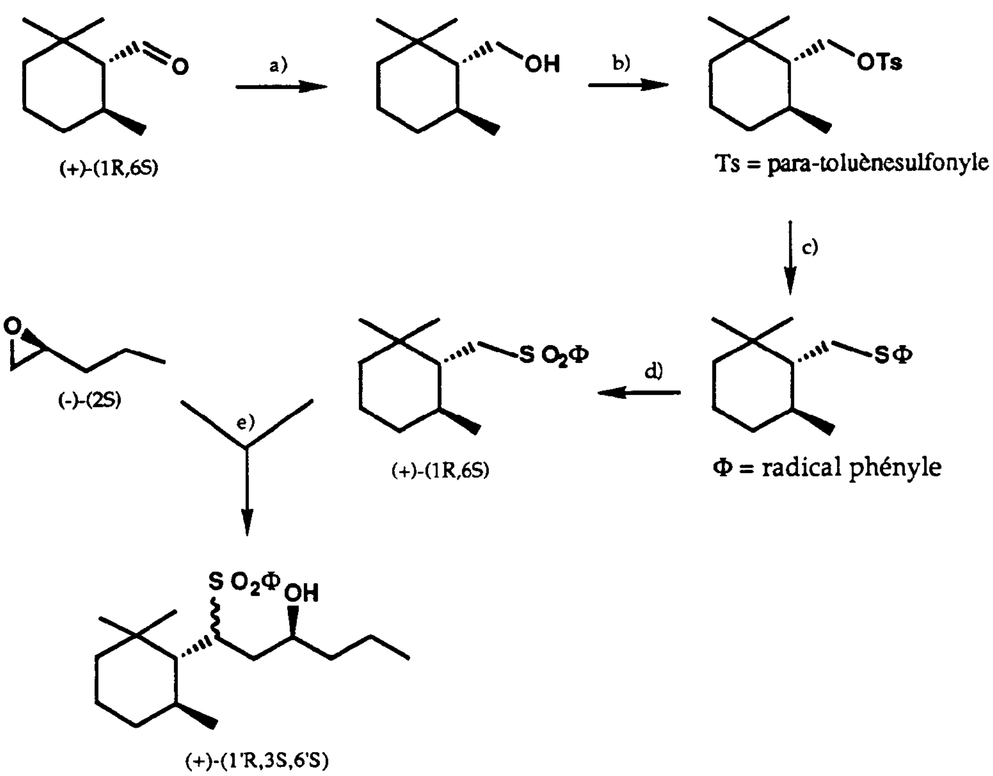


a) $NaBH_4$
b) Chlorure de para-toluènesulfonyle/pyridine
c) $C_6H_5SH$/K/EtOH

d) Acide m-chloropéroxybenzoïque (MCPBA)

e) n-Butyllithium/THF

La première étape de ce procédé est une réduction de l'énantiomère approprié du trans-2,2,6-triméthyl-1-cyclohexanecarbaldéhyde en l'alcool correspondant. Cet alcool est ensuite transformé en une sulfone, en trois étapes, de façon analogue à celle décrite par R. J. Armstrong et al. dans la référence citée auparavant.

Afin de garantir la pureté optique du produit final désiré, les sulfones ainsi obtenues ont été recristallisées de l'éthanol à plusieurs reprises, jusqu'à obtenir une valeur constante de l'angle de rotation optique correspondant. L'analyse spectroscopique de ces sulfones [RMN, utilisant un "shift reagent" chiral, à savoir Eu-$(HFBC)_3$, HFBC = 3-(heptafluorobutyryl)-d-camphorato] a montré que leur pureté optique était supérieure à 99%.

De même, une pureté optique du même degré des deux énantiomères du 2-propyloxirane, préparés à partir de D- ou L-norvaline selon un procédé connu [voir par exemple B. Koppenhoefer et al., Synthesis 1982, 316], a été garantie par recristallisation répétée de produits intermédiaires dans le procédé. L'analyse spectroscopique, dans les conditions citées ci-dessus, des (+)-(2R)-2-propyloxirane et (-)-(2S)-2-propyloxirane a révélé, dans les deux cas, une pureté optique supérieure à 99%.

La dernière étape du procédé illustré au schéma II consiste en un couplage régiosélectif de l'un ou l'autre isomère optiquement pur de ladite sulfone à savoir, (+)-(1R,6S) ou (-)-(1S,6R), déprotoné au préalable à l'aide de n-butyllithium dans le THF, avec l'un ou l'autre des isomères optiquement actifs purs du 2-propyloxirane susmentionnés. Cette réaction permet d'obtenir les quatre isomères trans optiquement actifs purs de l'hydroxysulfone, dont un seul est représenté au schéma cité (sans considération de l'isomérie du groupe $SO_2\Phi$).

Le procédé décrit ci-dessus pour la préparation des composés de formules (IIa) à (IId) selon l'invention peut, bien entendu, être employé de façon analogue pour préparer les épimères en C(3) des composés de formule (Ic) ou (Id). Il suffit pour autant d'utiliser en tant que produit de départ, dans le procédé représenté au schéma II, un isomère optiquement actif approprié du 2,2,c-3,t-6-tétraméthyl-r-1-cyclohexanecarbaldéhyde. Comme il a été cité précédemment, ledit isomère peut être obtenu comme il est décrit dans la demande de brevet européen publiée sous le n° 374509. D'autre part, les homologues de chaîne méthylée des composés (IIa-IId) peuvent être obtenus comme illustré au schéma II, en remplaçant le 2-propyloxyrane par le 2-(2'-méthylpropyl)oxirane dans la dernière étape représentée, accessible à partir de D- ou L-leucine.

L'invention sera maintenant décrite de façon plus détaillée à l'aide des exemples de préparation présentés ci-après, dans lesquels les températures sont indiquées en degrés centigrades et les abréviations ont le sens usuel dans l'art. L'invention sera également illustrée par des exemples d'application en parfumerie.

Exemple 1

Préparation de (+)-(1'R,3'S,6'S)-1-(2',2',3',6'-tétraméthyl-1'-cyclohexyl)-3-hexanol

a) (+)-(1'R,3'S,6'S)-1-(2',2',3',6'-tétraméthyl-1'-cyclohexyl)-3-hexanone

Dans un réacteur sous azote on a introduit 15,3 g (91,07 mmole) de (+)-(3S,5S,8S)-4,4,5,8-tétraméthyl-1-oxaspiro[2.5]octane ($[\alpha]^D_{20}$ = +36,0°; préparé comme décrit dans la demande de brevet européen No. 374 509) dans 200 ml de toluène, en présence de 2 ml d'éthérate de $BF_3$, et on a brassé le tout à température ambiante pendant 15 min. Le mélange de la réaction a été versé sur glace, extrait à l'éther, lavé à neutralité à l'aide de NaCl aqueux, séché sur $Na_2SO_4$, filtré et concentré. On a obtenu environ 15,3 g d'un produit brut, identifié par chromatographie en phase gazeuse comme étant (+)-(1R,3S,6S)-2,2,3,6-tétraméthyl-1-cyclohexanecarbaldéhyde. Ce produit a été utilisé tel quel dans la poursuite de la synthèse ($[\alpha]^D_{20}$ = +7,4°, c=2,5% $CHCl_3$).

Dans un ballon tricol muni d'un réfrigérant, d'un thermomètre et d'une ampoule à introduire, maintenu sous azote, on a introduit 2,30 g (0,100 at. g) de Na dans 35 ml d'éthanol (sec) et on a brassé jusqu'à formation complète de l'éthylate. On a ensuite refroidi à 0° à l'aide d'un bain glacé, introduit goutte à goutte une solution de 15,3g (91 mmole) de l'aldéhyde susmentionné et de 23,2g (0,270 mole) de 2-pentanone et laissé brasser pendant la nuit à température ambiante. Le mélange de la réaction a été versé sur glace, lavé à neutralité, séché sur $Na_2SO_4$, filtré et concentré. Après distillation au four à boules on a obtenu environ 3,6g (rend. 17% à partir de l'époxyde) de (+)-(1'R,3'S,6'S)-1-(2',2',3',6'-tétraméthyl-1'-cyclohexyl)-1-hexén-3-one pure ($[\alpha]^D_{20}$ = +19,0°).

Dans un ballon à 1 col muni d'une tête à hydrogénation, on a introduit 3,35 g (14,19 mmole) de l'énone préparée ci-dessus, 35 ml de cyclohexane, 500 mg de Pd/C à 5% et hydrogéné le mélange pendant 1 nuit (volume de $H_2$ consommé = 210 ml) à température ambiante. On a filtré sur Celite ® et concentré pour obtenir 3,8g de produit brut. Après distillation au four à boules ($0,05x10^2$ Pa) on a obtenu 3,0 g de (+)-(1'R,3'S,6'S)-1-(2',2',3',6'-tétraméthyl-1'-cyclohexyl)-3-hexanone à l'état pur (rend. 89%).

$[\alpha]^D_{20}$ = +8,8°

Les données analytiques de ce produit étaient identiques aux valeurs publiées par K. H. Schulte-Elte et al. pour l'hexanone racémique, ou 1-(2,2,c-3,t-6-tétraméthyl-r-1-cyclohexyl)-3-hexanone, dans Helv. Chim. Acta 68, 1976 (1985).

b) Dans un ballon à 4 cols, muni d'une agitation mécanique, on a chargé 10 ml d'éther sulfurique absolu et 0,15 g (3,15 mmole) de $LiAlH_4$. On a introduit goutte à goutte une solution de 1,0 g (4,2 mmole) de la cétone préparée selon a) dans 10 ml d'éther. Le mélange a été maintenu sous agitation, à température ambiante, pendant une nuit. On a hydrolysé le produit de la réaction à l'aide de 0,15 ml d'eau, 0,15 ml de NaOH 4N et 0,45 ml d'eau. Après avoir filtré sur Celite ® et concentré, on a obtenu 1,3 g de produit brut. La distillation au four à boules de ce dernier (0,05 x $10^2$ Pa) a fourni 0,9 g (rend. 90%) de (+)-(1′R,3′S,6′S)-1-(2′,2′,3′,6′-tétraméthyl-1′-cyclohexyl)-3-hexanol pur.

$[\alpha]^D_{20}$ = +10,6°

Les données analytiques de ce composé étaient identiques à celles du 1-(2,2,c-3,t-6-tétraméthyl-r-1-cyclohexyl)-3-hexanol publiées dans la référence citée sous a). La pureté optique (> 98%) de la cétone de formule (IIId) de départ a été confirmée par RMN en présence de $Eu(HFBC)_3$.

Exemple 2

Préparation de (-)-(1′S,3′R,6′R)-1-(2′,2′,3′,6′-tétraméthyl-1′-cyclohexyl)-3-hexanol

Ce composé a été préparé de façon analogue à celle décrite dans l'exemple 1, mais en partant de 11,3 g (66,66 mmole) de (-)-(3R,5R,8R)-4,4,5,8-tétraméthyl-1-oxaspiro[2.5]octane ($[\alpha]^D_{20}$ = -35,1° ; préparé comme décrit dans la demande de brevet européen No. 374509).

On a obtenu 3,4 g de (-)-(1′S,3′R,6′R )-1-(2′,2′,3′,6′-tétraméthyl-1′-cyclohexyl)-1-hexén-3-one : $[\alpha]^D_{20}$ = -23,0°. L'hydrogénation de 3,3 g de l'énone susmentionnée a fourni 3,2 g de (-)-(1′S,3′R, 6′R)-1-(2′,2′,3′,6′-tétraméthyl-1′-cyclohexyl)-3-hexanone pure : $[\alpha]^D_{20}$ = -8,6°.

Le traitement de 3,2 g de cette cétone, selon la méthode décrite dans l'exemple 1b), a fourni 2,8 g de (-)-(1′S,3′R,6′R)-1-(2′,2′,3′,6′-tétraméthyl-1′-cyclohexyl)-3-hexanol pur : $[\alpha]^D_{20}$ = -10,4°.

La pureté optique (> 98%) de la cétone de formule (IIIc) de départ a été confirmée par RMN en présence de $Eu(HFBC)_3$.

Exemple 3

Préparation de (+)-(1′R,3S,6′S)-1(2′,2′,6′-triméthyl-1′-cyclohexyl)-3-hexanol

a) (+)-(1R,6S) phényl (2,2,6-triméthyl-1-cyclohexyl)méthyl sulfone

Dans un réacteur contenant 5,0 g (0,13 mole) de $NaBH_4$ dans 150 ml d'éthanol on a ajouté 19,2 g (0,10 mole, 83% pur) de (+)-(1R,6S)-2,2,6-triméthyl-1-cyclohexanecarbaldéhyde ($[\alpha]^D_{25}$ = +0,68° ; mélange 1R:1S = 8:1; préparé selon le procédé décrit dans EP 255 904). Après 3 h de brassage, le mélange de la réaction a été versé sur de l'eau et extrait à l'éther de pétroleum (30-60°). On a combiné les extraits et séché sur $Na_2SO_4$ à pression réduite. L'alcool brut ainsi obtenu, 20,3 g, a été dissous dans 200 ml de pyridine et on a ajouté, en 5 portions, 27,4 g (0,14 mole) de chlorure de p-toluènesulfonyle. Après brassage pendant la nuit, on a versé le mélange sur l'eau froide et extrait à l'éther. On a lavé les extraits combinés avec une solution aqueuse à 10% de HCl, une solution aqueuse de $NaHCO_3$ et NaCl aqueux. On a séché sur $Na_2SO_4$ et concentré à pression réduite. Le tosylate brut ainsi obtenu, 29 g, a été dissous dans 50 ml d'éthanol et ajouté, goutte à goutte, à une solution de 7,7 g (0,20 mole) de potassium et 21,5g (0,20 mole) de thiophénol dans 450 ml d'éthanol, à 0°. Le mélange réactionnel a été brassé pendant la nuit à température ambiante et ensuite versé sur $H_2O$ et extrait à l'éther. Les extraits combinés ont été lavés avec NaOH aqueux à 10%, $NH_4Cl$ aqueux, $NaHCO_3$ aqueux et NaCl aqueux. On a séché sur $Na_2SO_4$ et évaporé le solvant à pression réduite. Distillation sur colonne Vigreux de 10 cm a fourni 16,5 g de (-)-(1R,6S)-2,2,6-triméthyl-1-(phénylthiométhyl)cyclohexane.

P. eb. 117-119°/0,9x$10^2$ Pa ; rend. 64%

$[\alpha]^D_{20}$ = -11,1°

IR : 3010, 2900, 1580, 1475, 1360, 1167, 1018, 945, 725 $cm^{-1}$

RMN($^1H$, 360MHz, $CDCl_3$) : 0,88(s, 3H) ; 0,93(s, 3H) ; 1,01(d, J=7,7Hz, 3H) ; 0,96-1,08(m, 1H) ; 1,19(m, 1H) ; 1,32-1,61(m, 5H) ; 1,67(m, 1H) ; 2,76(dd, J=5,4, 11,0Hz, 1H) ; 3,01(dd, J=3,5, 11,0Hz, 1H) ; 7,16(m, 1H) ; 7,29(m, 4H) δ ppm.

SM : 240(29, M+), 138(10), 123(55), 109(100), 95(35), 83(33), 69(43), 55(26),

41(30).

A une solution de 16,5 g (66,5 mmole) du thioéther préparé ci-dessus dans 250 ml de $CH_2Cl_2$, à 0°, on a ajouté lentement 28 g (0,16 mole) de MCPBA (acide m-chloropéroxybenzoïque). On a enlevé le bain de glace et laissé le mélange sous brassage pendant la nuit. La réaction a été arrétée avec $H_2O$ et le mélange extrait à l'éther. La phase organique a été lavée deux fois avec $NaHCO_3$ aqueux et une fois avec NaCl aqueux. On a séché sur $Na_2SO_4$ et concentré pour obtenir une huile visqueuse. Cette dernière a cristallisé lorsque laissée au repos pour fournir 18,5 g (rend. 99%) de phényl (2,2,6-triméthyl-1-cyclohexyl)méthyl sulfone. Ce produit a été recristallisé 3 fois dans l'éthanol pour fournir un seul énantiomère pur, à savoir le(+)-(1R,6S).

P. f. 73°

$[a]^D_{25}$ = +23,71° (toluène, c=39,308 g/100 ml)

IR : 3112, 2900, 1580, 1440, 1295, 1168, 1143, 975, 760 $cm^{-1}$

RMN($^1$H, 360MHz, $CDCl_3$) : 0,72(s, 3H) ; 0,83(s, 3H) ; 0,94(d, J=7Hz, 3H) ; 1,02(m, 1H) ; 1,52-1,99(m, 5H) ; 1,61(m, 1H) ; 1,70(m, 1H) ; 2,94(dd, J=4, 15Hz, 1H) ; 3,11(dd, J=3,5, 15Hz, 1H) ; 7,56(t, J=7Hz, 2H) ; 7,64(t, J=7Hz, 1H) ; 7,93(d, J=7Hz, 1H) δ ppm.

SM : 280(1, M+), 169(3), 138(83), 123(48), 109(10), 95(39), 83(52), 77(100), 69(42), 55(43), 51(18), 41(39).

b) (-)-(2S)-2-propyloxirane

Dans un ballon on a dissous 48,5 g (0,41 mole) de D-norvaline dans 700 ml de HCl (6N) et refroidi la solution à -8°. On a ajouté, sous agitation vigoureuse, 46,4 g (0,67 mole) de $NaNO_2$, en maintenant la température de la réaction entre -8° et -5°. Après 15 h de brassage à -5°, on a extrait le mélange réactionnel à l'éther. Les extraits ont été combinés et lavés avec une solution aqueuse saturée en NaCl, ensuite séchés sur $Na_2SO_4$ et concentrés. On a obtenu 48,1 g de produit brut qui a été dissous dans 50 ml d'éther et ajouté goutte à goutte à une suspension de 12,5 g (0,33 mole) de $LiAlH_4$ dans 200 ml d'éther, à -10°. L'excès de $LiAlH_4$ a été hydrolysé avec 30 ml d'eau, et 250 ml de $H_2SO_4$ aqueux à 10% ont été ajoutés pour dissoudre les sels d'aluminium résultants. On a séparé la phase organique, saturé la phase aqueuse avec NaCl et extrait 2 fois à l'éther. Les extraits organiques combinés ont été séchés sur $Na_2SO_4$ et concentrés à pression atmosphérique. On a distillé sur une colonne Vigreux de 15 cm pour obtenir 27,3g de 2-chloro-1-pentanol (P.f. 66-69°; rend. 53%).

A une solution de 26,0g (0,21 mole) du chloropentanol ainsi obtenu et 33 g (0,42 mole) de pyridine dans 500 ml de toluène, à 0°, on a ajouté, par portions, 57,5 g (0,25 mole) de chlorure de 3,5-dinitrobenzoyle. Après brassage pendant la nuit, on a versé le mélange sur $H_2O$ et extrait au toluène. Les extraits combinés ont été lavés avec HCl aqueux à 10% et des solutions aqueuses saturées en $NaHCO_3$ et NaCl. On a séché sur $Na_2SO_4$ et concentré à pression réduite. Le chlorodinitrobenzoate brut ainsi formé a été recristallisé 5 fois dans un mélange d'éther isopropylique/éthanol 2:1, pour fournir 26 g de produit pur [1 seul énantiomère, configuration (2R)]. A un mélange de 24,0 g (75,8 ml) de ce produit et 250 ml de méthanol on a ajouté 1 ml de $CH_3ONa$ à 30% dans le méthanol. Après 1 h, la solution rose a été versée sur NaCl aqueux saturé et extraite 3 fois à l'éther. On a lavé les extraits avec NaCl saturé, séché ($Na_2SO_4$) et concentré à pression atmosphérique. Après distillation on a obtenu 8,1 g de (2R)-2-chloro-1-pentanol, dont 6,94 g (56,6 mole) ont été refroidis à 0°. On a ensuite ajouté 6,4g (0,11 mole) de KOH fraîchement moulu. On a brassé à température ambiante pendant 1 h et distillé au four à boules (pression atmosphérique). Une distillation supplémentaire sur $CaH_2$ a fourni 4,7g de (-)-(2S)-2-propyloxirane (rend. 96% à partir du chloropentanol).

P. eb. 90° (bain)

$[\alpha]^D_{25}$ = -15,8°

IR : 2925, 1455, 1403, 1378, 1255, 1130 (large) $cm^{-1}$

RMN($^1$H, 360MHz, $CDCl_3$) : 0,98(m, 3H) ; 1,51(m, 4H) ; 2,47(dd, J=3,5Hz, 1H) ; 2,75(t, J=3Hz, 1H) ; 2,91(m, 1H) δ ppm.

SM : 86(0, M+), 71(71), 55(15), 43(16), 41(100), 39(32).

c) (1′R,3S,6′S)-1-phénylsulfonyl-1-(2′,2′,6′-triméthyl-1′-cyclohexyl)-3-hexanol

On a dissous 4,8 g (17,1 mmole) de la sulfone obtenue selon a) dans 150 ml de THF, refroidi à -30°, et ajouté goutte à goutte 11 ml de n-butyllithium (1,68N). La température de la réaction a été maintenue entre -30° et -40° pendant 2,5 h et ensuite baissée à -78°. On a ajouté au mélange réactionnel 2,0 g (20,8 mmole) de (-)-(2S)-2-propyloxirane, préparé selon b), dans 17 ml de HMPA (hexaméthylphosphoramide) et, ensuite, goutte à goutte, 3,0 ml de $BF_3$-$(C_2H_5)_2O$. Après brassage pendant 5h à -78°, on a laissé chauffer lentement pendant la nuit, jusqu'à température ambiante. Le mélange réactionnel a été versé sur $H_2O$ et extrait à l'éther. L'extrait a été lavé avec une solution aqueuse saturée de $NaHCO_3$ et une solution

aqueuse saturée de NaCl. On a séché sur $Na_2SO_4$ et concentré à pression réduite. Le produit a été soumis à chromatographie sur colonne de silica gel, en utilisant comme éluant du toluène, un mélange d'acétate d'éthyle-toluène à 2% et un mélange d'acétate d'éthyle-toluène à 4%, pour fournir l'hydroxy-sulfone désirée, sous forme d'un mélange de diastéréomères [rend. 65% à partir de la sulfone préparée en a)].

IR : 3450, 2920, 1455, 1443, 1290, 1135, 1180 $cm^{-1}$

RMN($^1H$, 360MHz, $CDCl_3$) : mélange de deux diastéréomères) : 0,33(s) ; 0,73(s) ; 3,26(d, J=7Hz) ; 3,61(m) ; 3,74(m, absent avec $D_2O$) ; 7,50-7,68(m) ; 7,92(dd, J=7, 16Hz) δ ppm.

SM : 366(0, $M^+$), 225(20), 207(21), 137(32), 123(39), 109(39), 95(41), 81(49), 77(25), 73(51), 69(93), 55(100), 41(87).

d) 3,7g (10 mmole) de l'hydroxy-sulfone préparée selon c) ont été dissous dans 100 ml de THF et la solution refroidie à -78°. On a ajouté goutte à goutte du naphtalure de lithium (0,7 M/THF) jusqu'à obtenir une couleur verte persistante (40 ml). Après 10 min., on a ajouté 20 ml d'éthanol et laissé la température monter jusqu'à température ambiante. Le mélange de la réaction a été versé sur $H_2O$ et extrait à l'hexane. Les extraits ont été lavés avec une solution aqueuse à 10% de NaOH, une solution aqueuse à 10% de HCl et une solution aqueuse saturée en NaCl. On a séché sur $Na_2SO_4$ et concentré. Le produit brut à été filtré pour éliminer le naphtalène ($SiO_2$, 50 g ; éluant : hexane, ensuite éther) et ensuite distillé au four à boules (190°/0,5x$10^2$ Pa) pour fournir 1,6 g d'un mélange brut contenant l'alcool désiré et son homologue à chaîne saturée, dans une proportion de 5,5:1.

Ce mélange brut a été traité avec 10 ml d'anhydride acétique (11 g ; 0,11 mole), 3 ml de triéthylamine (2,1 g ; 0,02 mole) et 50 mg de diméthylaminopyridine pendant 1 h, à une température de 0 à 5°. Après avoir versé sur de la glace et extrait à l'hexane, on a lavé les extraits à l'eau, $NaHCO_3$ et NaCl, séché sur $Na_2SO_4$ et concentré. Le résidu a été dissous dans 25 ml d'hexane, refroidi à -78° et saturé à l'ozone jusqu'à ce que la couleur bleue persiste pendant 30 min. La solution a été dégazée avec de l'azote, chauffée à 0° et ajoutée, sur une période de 10 min., à une bouillie de 1,0 g (26 mmole) de $LiAlH_4$ dans 20 ml d'éther. On a laissé la température monter à reflux et continué à brasser pendant 1 h. Après avoir hydrolysé avec 5 ml de NaOH à 3%, filtré le précipité blanc, séparé la phase aqueuse et concentré, on a obtenu 0,85 g de l'hexanol désiré sans aucune trace de son homologue de chaîne saturée. Ce produit a été soigneusement purifié sur une colonne de silice Merck "Lobar C", en utilisant comme éluant un mélange d'hexane/acétate d'éthyle 39:1. On a selectionné les fractions les plus pures qui ont été distillées au four à boules (200°/0,5x$10^2$ Pa) pour fournir 0,41 g (rend. 18% à partir de l'hydroxy-sulfone) de (+)-(1'R,3S,6'S)-1-(2',2',6'-triméthyl-1'cyclohexyl)-3-hexanol d'une pureté supérieure à 99%. Les données analytiques de ce composé étaient les suivantes :

$[\alpha]^D_{20}$ = +12,6° (éthanol, c=10,0 g/100 ml)

IR : 3310, 2860, 1450, 1362, 994 $cm^{-1}$

RMN($^1H$, 360MHz, $CDCl_3$) : 0,50(m, 1H) ; 0,79(s, 3H) ; 0,89(s, 3H) ; 0,89(d, J=6,8Hz, 3H) ; 0,93(t, J=6,5Hz, 3H) ; 1,00-1,20(m, 2H) ; 1,20-1,70(m, 14H) ; 3,55(s large, 1H) δ ppm.

SM : 226(0, $M^+$), 208(15), 193(28), 183(12), 165(12), 152(11), 138(28), 124(45), 109(57), 95(43), 82(45), 69(68), 55(100), 41(88).

Exemple 4

Préparation de (+)-(1'R,3R,6'S)-1-(2',2',6'-triméthyl-1'-cyclohexyl)-3-hexanol

a) (+)-(2R)-2-propyloxirane

Ce composé a été préparé à partir de L-norvaline de la même façon que celle décrite dans l'exemple 3b). Ses données analytiques étaient identiques à celles de son antipode y décrit, à l'exception de $[\alpha]^D_{25}$ = +16,0°

b) (1'R,3R,6'S)-1-phénylsulfonyl-1-(2',2',6'-triméthyl-1'-cyclohexyl)-3-hexanol

Cette hydroxy-sulfone a été préparée selon la méthode décrite dans l'exemple 3c), à partir de la sulfone obtenue selon l'exemple 3a) et de (+)-(2R)-2-propyloxirane préparé comme décrit ci-dessus.

On a utilisé 5,0 g (17,8 mmole) de (+)-(1R,6S) phényl (2,2,6-triméthyl-1-cyclohexyl)méthyl sulfone, 3,4 g (39,5 mmole) de (+)-(2R)-2-propyloxirane, 12 ml de n-butyllithium (1,68N) et 4,5 ml de $BF_3$-$(C_2H_5)_2O$. On a obtenu (1'R,3R,6'S)-1-phénylsulfonyl-1-(2',2',6'triméthyl-1'-cyclohexyl)-3-hexanol (mélange de deux diastéréomères) avec un rendement de 61% calculé sur la sulfone de départ.

IR : 3500, 2925, 1450, 1295, 1143, 1023 $cm^{-1}$

RMN($^1H$, 360MHz, $CDCl_3$) : 0,78(s) ; 0,86(s) ; 1,03(d, J=7Hz:) ; 3,46(m) ; 3,65-3,80(m) ; 4,01(d,

J=8HZ) ; 7,15-7,28(m) ; 7,50-7,66(m) ; 7,91(d, J=7Hz) δ ppm.

SM : 366(0, $M^+$), 225(9), 207(12), 137(19), 123(24), 109(23), 95(31), 81(35), 73(31), 69(69), 55(100), 41(93).

c) On a procédé selon l'exemple 3d), à partir de l'hydroxy-sulfone obtenue selon b). On a obtenu 700 mg (rend. 29% à partir de l'hydroxy-sulfone) de (+)-(1'R,3R,6'S)-1-(2',2',6'-triméthyl-1'-cyclohexyl)-3-hexanol d'une pureté supérieure à 99%.

$[\alpha]^D_{20}$ = +12,1° (éthanol, c=10,0 g/100 ml)

IR : 3310, 2860, 1450, 1362, 1120, 998 $cm^{-1}$

RMN($^1H$, 360MHz, $CDCl_3$) : 0,50(m, 1H) ; 0,79(s, 3H) ; 0,89(s, 3H) ; 0,89(d, J=6,5Hz, 3H) ; 0,95(t, J=7,2Hz, 3H) ; 1,10-1,30(m, 2H) ; 1,30-1,70(m, 14H) ; 3,65(s large, 1H) δ ppm.

SM : 226(0, $M^+$), 208(13), 193(23), 183(13), 165(11), 152(10), 138(26), 123(43), 109(52), 95(44), 82(40), 69(73), 55(100), 41(85).

Exemple 5

Préparation de (-)-(1'S,3S,6'R)-1-(2',2',6'-triméthyl-1'-cyclohexyl)-3-hexanol

a) (-)-(1S,6R) phényl (2,2,6-triméthyl-1-cyclohexyl)méthyl sulfone

Cette sulfone a été préparée par une méthode analogue à celle décrite dans l'exemple 3a) mais à partir de (-)-(1S,6R)-2,2,6-triméthyl-1-cyclohexane-carbaldéhyde ($[\alpha]^D_{25}$ = -0,80° ; mélange 1S:1R = 9:1 ; préparé selon le procédé décrit dans EP 255 904). On a obtenu (+)-(1S,6R)-2,2,6-triméthyl-1-(phénylthiométhyl)-cyclohexane avec un rendement de 62% et les données suivantes :

P. eb. 110-111°/0,65x$10^2$ Pa

$[\alpha]^D_{25}$ = +12,4°

Les autres données étaient identiques à celles de l'antipode décrit dans l'exemple 3a).

Ce thioéther a ensuite été transformé comme décrit précédemment, pour fournir, avec 99% de rendement, la sulfone susmentionnée. Cette dernière a été recristallisée 5 fois dans l'éthanol pour fournir un seul énantiomère pur, à savoir la (-)-(1S,6R) phényl (2,2,6-triméthyl-2-cyclohexyl)méthyl sulfone.

P.f. 79,5°

$[\alpha]^D_{25}$ = -24,55° (toluène, c=35,028 g/100 ml)

Les autres données analytiques étaient identiques à celles décrites dans l'exemple 3a) pour l'antipode.

b) (1'S,3S,6'R)-1-phénylsulfonyl-(2',2',6'-triméthyl-1'-cyclohexyl)-3-hexanol

Obtenu avec 66% de rendement à partir de la sulfone décrite sous a) et de (-)-(2S)-2-propyloxirane [exemple 3b)], selon le procédé décrit dans l'exemple 3c).

On a utilisé 5,0 g (17,8 mmole) de (-)-(1S,6R) phényl (2,2,6-triméthyl-1-cyclohexyl)méthyl sulfone, 2,3 g (26,7 mmole) de (-)-(2S)-2-propyloxirane, 12 ml de n-butyllithium (1,68N) et 3,2 ml de $BF_3$-$(C_2H_5)_2O$.

On a obtenu l'hydroxy-sulfone en titre sous forme d'un mélange de 2 diastéréomères.

IR : 3400 (large), 2910, 1458, 1445, 1380, 1292, 1135, 1075, 1015 $cm^{-1}$

RMN($^1H$, 360MHz, $CDCl_3$) : 0,78(s) ; 0,86(s) ; 1,03(d, J=7Hz) ; 3,46(m) ; 3,65-3,80(m) ; 4,10(d, J=8Hz) ; 7,13-7,28(m) ; 7,50-7,66(m) ; 7,91(d, J=7Hz) δ ppm.

SM : 366(0, $M^+$), 225(9), 207(11), 137(19), 123(23), 109(23), 95(33), 81(36), 73(31), 69(70), 55(100), 41(98).

c) On a procédé selon l'exemple 3d) en utilisant comme produit de départ l'hydroxy-sulfone préparée en b). On a obtenu 450 mg (rend. 21% à partir de l'hydroxy-sulfone) de (-)-(1'S,3S,6'R)-1-(2',2',6'-triméthyl-1'-cyclohexyl)-3-hexanol à plus de 99% pur.

$[\alpha]^D_{20}$ = -11,6° (éthanol, c=10,1 g/100 ml)

Les autres données analytiques de ce composé sont identiques à celles de son énantiomère faisant l'objet de l'exemple 4.

Exemple 6

Préparation de (-)-(1'S,3R,6'R)-1-(2',2',6'-triméthyl-1'-cyclohexyl)-3-hexanol

a) (1'S,3R,6'R)-1-phénylsulfonyl-(2',2',6'-triméthyl-1'-cyclohexyl)-3-hexanol

Cette hydroxy-sulfone a été préparée selon la méthode décrite dans l'exemple 3c) à partir de la sulfone obtenue dans l'exemple 5a) et de (+)-(2R)-2-propyloxirane obtenu selon l'exemple 4a).

On a utilisé 5,0 g (17,8 mmole) de (-)-(1S,6R) phényl (2,2,6-triméthyl-1-cyclohexyl)méthyl sulfone, 3,4 g (39,5 mmole) de (+)-(2R)-2-propyloxirane, 12 ml de n-butyllithium (1,68N) et 4,5 ml de $BF_3$-$(C_2H_5)_2O$. On a obtenu (1′S,3R,6′R)-1-phénylsulfonyl-(2′,2′,6′-triméthyl-1′-cyclohexyl)-3-hexanol (mélange de 2 diastéréomères) avec un rendement de 74% calculé sur la sulfone de départ.

IR : 3450 (large), 2940, 1460, 1445, 1300, 1140, 1080 $cm^{-1}$

RMN($^1$H, 360MHz, $CDCl_3$) : 0,33(s) ; 0,73(s) ; 3,28(d, J=7Hz) ; 3,62(m) ; 7,13-7,29(m) ; 7,50-7,68(m) ; 7,91(dd, J=7,16Hz) δ ppm.

SM : 366(0, $M^+$), 225(7), 207(9), 137(16), 123(20), 109(20), 81(34), 73(33), 69(69), 55(100), 41(97).

b) On a procédé selon l'exemple 3d) à partir de l'hydroxy-sulfone préparée selon a). On a obtenu 410 mg (rend. 14% à partir de l'hydroxy-sulfone) de (-)-(1′S,3R,6′R)-1-(2′,2′,6′-triméthyl-1′-cyclohexyl)-3-hexanol à plus de 99% pur.

$[\alpha]^D_{20}$ = -11,1° (éthanol, c=9,9 g/100 ml)

Les autres données analytiques étaient identiques à celles de l'énantiomère décrit dans l'exemple 3.

## Exemple 7

### Préparation de (+)-(1′R,6′S)-5-méthyl-1-(2′,2′,6′-triméthyl-1′-cyclohexyl)-3-hexanol

Ce composé a été préparé selon la méthode décrite dans l'exemple 1, à partir de (+)-(1R,6S)-2,2,6-triméthyl-1-cyclohexanecarbaldéhyde (5 g, 32 mmole ; obtenu selon la méthode décrite dans EP 255 904), 20 ml d'éthanol, 0,92 g (0,04 at. g) de Na coupé en carrelets, et 5 g (50 mmole) de 4-méthyl-2-pentanone. Après le traitement décrit et distillation au four à boules (150°/13,3 Pa) on a obtenu 4,94 g (rend. 65%) de produit contenant la (+)-(1′R,6′S)-5-méthyl-1-(2′,2′,6′-triméthyl-1′-cyclohexyl)-1-hexén-3-one, en mélange avec son isomère (1′S,6′S) dans un rapport 92:8.

$[\alpha]^D_{20}$ = +16,1°

IR : 1700, 1675, 1625, 1000 $cm^{-1}$

RMN($^1$H, 360MHz) : 0,75(d, J=6,5Hz, 3H) ; 0,82 et 0,895(2s, 2x3H) ; 0,95(d, J=7,2Hz, 6H) ; 2,16(m, 1H) ; 2,415(d, J=7,2Hz, 2H) ; 6,48(d, J=15Hz, 1H) ; 5,72(dxd, J=10Hz et 15Hz, 1H) δ ppm.

SM : 236(8, $M^+$), 221(8), 193(24), 179(32), 161(15), 151(45), 136(52), 124(26), 109(60), 95(100), 85(68), 81(58), 69(58), 55(70), 41(58).

3 g de ce mélange ont ensuite été hydrogénés dans 30 ml d'éthanol et en présence de 0,3 g de nickel de Raney (température ambiante et pression atmosphérique). Après filtration et distillation au four à boules (150°/13,3 Pa), on a obtenu 2,7g de produit contenant la (+)-(1′R,6′S)-5-méthyl-1-(2′,2′,6′-triméthyl-1′-cyclohexyl)-3-hexanone (92%) et 8% de son isomère (1′S,6′S) (rend. 93%).

$[\alpha]^D_{20}$= +6,6°

IR : 1715 $cm^{-1}$ (C=0)

RMN($^1$H, 360MHz) : 0,54(ddd, J=2,4Hz, 5,2Hz, 11Hz, 1H) ; 0,8 et 0,874(2s, 2x3H) ; 0,78(d, J=6,5Hz, 3H) ; 0,91(d, J=7,2Hz, 6H) ; 2,13(m, 1H) ; 2,26(d, J=7,2Hz, 2H) ; 2,3-2,53(m, 2H) δ ppm.

SM : 238(12, $M^+$), 223(4), 205(4), 195(2), 181(22), 163(22), 153(7), 138(45), 123(30), 100(16), 95(40), 85(68), 69(70), 57(100), 41(46).

Ce mélange a ensuite été réduit comme il est décrit dans l'exemple 1b) en utilisant 0,19 g (5 mmole) de $LiAlH_4$, en suspension dans 15 ml d'éther (sec), et 23 g (9,7 mmole) de cétone dans 10 ml d'éther. On a laissé réagir à reflux pendant 2 h et ensuite traité avec 0,19 ml de $H_2O$, 0,19 ml de NaOH (15%) et 0,57 ml de $H_2O$. Le produit a été filtré, concentré et distillé au four à boules (150°/13,3 Pa) pour fournir 2,15 g de produit pur contenant 92% de (+)-(1′R,6′S)-5-méthyl-1-(2′,2′,6′-triméthyl-1′-cyclohexyl)-3-hexanol et 8% de son isomère (1′S,6′S).

$[\alpha]^D_{20}$ = +8,7°

IR : 3350 $cm^{-1}$

RMN($^1$H, 360MHz) : 0,526(m, 1H) ; 0,795 et 0,89(2s, 2x3H) ; 0,85-0,95(m, 3x3H) ; 3,61(m, 1H) δ ppm.

SM : 240(2, $M^+$), 222(4), 207(8), 183(5), 166(3), 151(2), 138(18), 123(28), 109(42), 95(40), 83(38), 69(100), 55(45), 41(40).

## Exemple 8

### Préparation de (-)-(1′S,6′R)-5-méthyl-1-(2′,2′,6′-triméthyl-1′-cyclohexyl)-3-hexanol

Ce composé a été préparé exactement de la même façon que celle décrite dans l'exemple 7, mais à partir

de (-)-(1S,6R)-2,2,6-triméthyl-1-cyclohexanecarbaldéhyde.
Les produits obtenus présentaient les mêmes données analytiques que leurs isomères décrits dans l'exemple 7, à l'exception de:
(-)-(1'S,6'R)-5-méthyl-1-(2',2',6'-triméthyl-1'-cyclohexyl)-1-hexén-3-one [mélange avec 8% (1'R,6'R), 2,62 g, rend. 35%]
$[\alpha]^{D}_{20}$ = -14,8°
(-)-(1'S,6'R)-5-méthyl-1-(2',2',6'-triméthyl-1'-cyclohexyl)-3-hexanone [mélange avec 8% (1'R,6'R), 2,8 g, rend. 95%]
$[\alpha]^{D}_{20}$ = -6,3°
(-)-(1'S,6'R)-5-méthyl-1-(2',2',6'-triméthyl-1'-cyclohexyl)-3-hexanol [mélange avec 8% (1'R,6'R), 2,1 g, rend. 92%]
$[\alpha]^{D}_{20}$ = -8,3°

Exemple 9

Préparation d'une composition parfumante

On a préparé une composition parfumante de base de type floral, musqué, aromatique par mélange des ingrédients suivants:

| Ingrédients | Parties en poids |
|---|---|
| Iralia ® 1) | 120 |
| Cyclosia ® 2) base | 100 |
| Exaltolide ® 3) à 10%* | 100 |
| Musc cétone | 80 |
| Musc xylol | 70 |
| Musc ambrette | 60 |
| Coumarine | 60 |
| Rosinol crist. | 50 |
| Essence de patchouli | 50 |
| Eugénol | 45 |
| Essence de galbanum résinoïde | 35 |
| Essence de rose blanche | 30 |
| Lilial ® 4) | 30 |
| Acétate de vétyvéryle | 30 |
| Méthyleugénol | 20 |
| Tricyclone 5) | 20 |
| β-ionone | 10 |
| Jasmin synthétique | 10 |
| Essence de résine benjoin | 10 |
| Essence d'orange déterpénée | 10 |
| Total | 940 |

* dans le dipropylèneglycol (DIPG)

1) méthylionone ; origine : Firmenich SA, Genève, Suisse

2) hydroxycitronellal ; origine : Firmenich SA, Genève, Suisse

3) cyclopentadécanolide ; origine : Firmenich SA, Genève, Suisse

4) 3-(4-tert-butyl-1-phényl)-2-méthylpropanal ; origine : L. Givaudan, Vernier, Suisse

5) 10,10-diméthyl-cis-tricyclo[7.1.1.0$^{2,7}$]undec-2-én-4-one (mélange d'isomères) ; origine : Firmenich SA, Genève, Suisse

Avec cette composition de base on a préparé cinq compositions A, B, C, D et E, avec les ingrédients suivants:

| Ingrédients | A | B | C | D | E |
|---|---|---|---|---|---|
| Composition de base | 940 | 940 | 940 | 940 | 940 |
| trans-1-(2,2,6-triméthyl-1-cyclohexyl)-3-hexanol (mélange racémique) à 10%* | 60 | --- | --- | --- | --- |
| (+)-(1'R,6'S)-1-(2',2',6'-triméthyl-1'-cyclohexyl)-3-hexanol (mélange épimères en C(3)) à 10%* | --- | 60 | --- | --- | --- |
| (-)-(1'S,6'R)-1-(2',2',6'-triméthyl-1'-cyclohexyl)-3-hexanol (mélange épimères en C(3)) | --- | --- | 60 | --- | --- |
| (+)-(1'R,3S,6'S)-1-(2',2',6'-triméthyl-1'-cyclohexyl)-3-hexanol à 10%* | --- | --- | --- | 60 | --- |
| (+)-(1'R,3'S,6'S)-1-(2',2',3',6'-tétraméthyl-1'-cyclohexyl)-3-hexanol à 1%* | --- | --- | --- | --- | 60 |
| Total | 1000 | 1000 | 1000 | 1000 | 1000 |

* dans le dipropylèneglycol (DIPG)

Les cinq compositions ainsi préparées ont été évaluées pour comparaison par un panel de six experts parfumeurs. De l'avis de ces derniers, lorsqu'on comparait les compositions nouvelles A, B et C avec la composition de base, on trouvait que l'odeur de cette dernière avait été enrichie d'une note boisée élégante et chaude, plus marquée et puissante dans le cas de la composition B que dans celui de la composition A, la note boisée de la composition C étant plus faible, en dépit du fait que la concentration d'ingrédient parfumant selon l'invention ajouté à cette composition était dix fois supérieure à celle des deux autres ingrédients parfumants ajoutés aux compositions A et C. Il ressortait, par ailleurs, de ces avis que la composition B était nettement préférée parmi les trois, son odeur étant non seulement plus puissante, mais aussi d'un caractère boisé plus net. Cet aspect se trouvait encore renforcé dans la composition D, lorsque celle-ci était comparée à la composition B.

Par ailleurs, lorsqu'on comparait les compositions D et E, contenant les deux composés préférés de l'invention, tous les parfumeurs étaient de l'avis que la composition E exhalait une odeur boisée plus forte que celle de la composition D, bien que la concentration de cette dernière en (+)-(1′R,3S,6′S)-1-(2′,2′,6′-triméthyl-1′-cyclohexyl)-3-hexanol ait été dix fois supérieure à la concentration de la composition E en (+)-(1′R,3′S,6′S)-1-(2′,2′,3′,6′-tétraméthyl-1′-cyclohexyl)-3-hexanol. Ce résultat rendait évidente la supériorité de la puissance olfactive de ce dernier ingrédient parfumant, mais la composition E n'a pas été pour autant unanimement préférée. En effet, plusieurs parfumeurs ont préféré le caractère boisé plus arrondi et élégant de la composition D, tandis que d'autres ont mieux apprécié la note boisée plus sèche et plus animale de la composition E. Ces résultats ont montré que les deux ingrédients parfumants selon l'invention se prêtaient à des applications en parfumerie parfaitement distinctes, leurs propriétés ne faisant pas double emploi.

Exemple 10

Préparation d'une composition parfumante pour un parfum de type masculin

On a préparé une composition parfumante de base par mélange des ingrédients suivants :

| Ingrédients | Parties en poids |
|---|---|
| Acétate de carbinol à 10%* | 200 |
| Acétate de géranyle | 50 |
| Acétate de linalyle | 1200 |
| Muscone | 100 |
| Cétone framboise à 10%* | 30 |
| Essence de bergamote naturelle | 300 |
| Essence de bergamote synthétique | 300 |
| Citral pur | 120 |
| Essence de citron | 1500 |
| Galaxolide ® 1) 50 | 250 |
| Exaltex ® 2) | 100 |
| Essence de clou de girofle | 300 |
| Essence de lavande | 600 |
| Linalol | 200 |
| Lyral ® 3) | 1000 |
| Essence de marjolaine douce | 350 |
| Absolue de mousse de chêne à 50%* | 150 |
| Essence de muscade | 500 |
| Salicylate de benzyle | 50 |
| Sandela ® 4) | 600 |
| Ylang | 300 |
| Essence de galbanum à 10%* | 300 |
| Total | 8500 |

* dans le dipropylèneglycol (DIPG)

1) 1,3,4,6,7,8-hexahydro-4,6,6,7,8,8-hexaméthylcyclopenta[g]isochromène ; origine : IFF Inc., USA

2) cyclopentadécanolide ; origine : Firmenich SA, Genève, Suisse

3) 4-(4-hydroxy-4-méthylpentyl)-3-cyclohexène-1-carboxaldéhyde ; origine : IFF Inc., USA

4) 3-(isocamphyl-5)-cyclohexan-1-ol ; origine : L. Givaudan, Vernier, Suisse.

Avec cette composition de base de type hespéridé, épicé, floral et vert, on a préparé quatre compositions parfumantes A, B, C et D, en ajoutant, respectivement, 1500 parties en poids de 1-(2,2,c-3,t-6-tétraméthyl-r-1-cyclohexyl)-3-hexanol à 1 %* (composition A), 1500 parties en poids de (+)-(1′R,3′S,6′S)-1-(2′,2′,3′,6′-tétraméthyl-1′-cyclohexyl)-3-hexanol à 1%* (composition B), 1500 parties en poids de (-)-(1′S,3′R,6′R)-1-(2′,2′,3′,6′-tétraméthyl-1′-cyclohexyl)-3-hexanol à 1%* (composition C) et 1500 parties en poids de (+)-(1′R,3S,6′S)-1-(2′,2′,6′-triméthyl-1′-cyclohexyl)-3-hexanol à 10%* (composition D).

Ces compositions ont été évaluées par un panel de parfumeurs qui a trouvé que, par rapport à la composition de base, la nouvelle composition B possédait une connotation boisée-ambrée, sèche et très perceptible, tandis que la composition C ne possédait aucun caractère boisé. Par ailleurs, lorsque comparée à la composition A, le caractère boisé-ambré de la composition était beaucoup plus prononcé, la composition A possédant également une note boisée plus faible.
En outre, de l'avis des parfumeurs, la note boisée de la composition D était plus arrondie et moins sèche que celle de la composition B, cette dernière exhalant une odeur plus animale.

## Revendications

**1.** Composé de formule

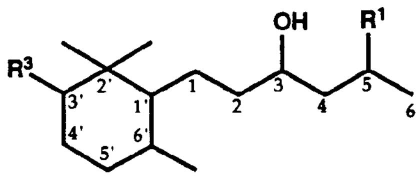

(I)

a) dans laquelle le symbole $R^3$ représente un atome d'hydrogène et le symbole $R^1$ représente un atome d'hydrogène ou un radical méthyle, sous forme d'un mélange optiquement actif d'épimères en C(3) de formule

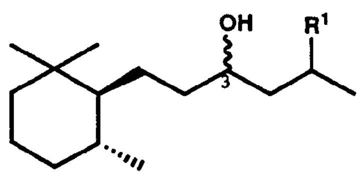

ou

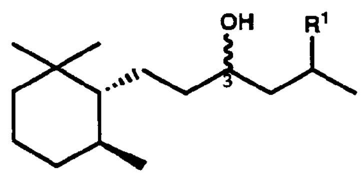

(-)-(1'S,6'R)
(Ia)

(+)-(1'R,6'S)
(Ib)

dans laquelle la ligne ondulée indique l'une ou l'autre des deux orientations possibles du groupe OH, ou sous forme de l'un des épimères en C(3) composant ledit mélange ; ou
b) dans laquelle le symbole $R^3$ représente un radical méthyle et le symbole $R^1$ représente un atome d'hydrogène, sous forme d'un mélange optiquement actif d'épimères en C(3) de formule

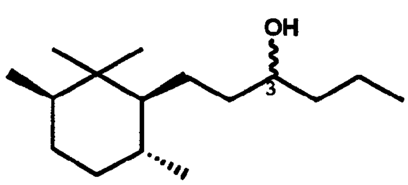

ou

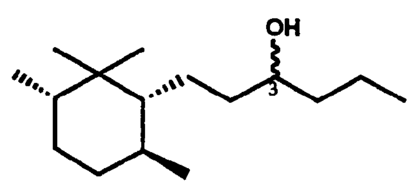

(-)-(1'S,3'R,6'R)
(Ic)

(+)-(1'R,3'S,6'S)
(Id)

dans laquelle la ligne ondulée a le sens indiqué ci-dessus, ou sous forme de l'un des épimères en C(3) composant ledit mélange.

**2.** A titre de composé selon la revendication 1, l'un des diastéréomères optiquement actifs de formule

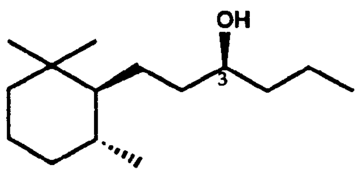

ou

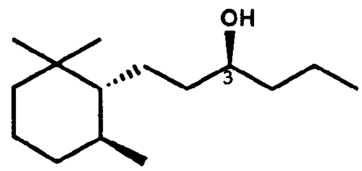

(-)-(1'S,3S,6'R)
(IIa)

(+)-(1'R,3S,6'S)
(IIb)

ou son épimère en C(3).

3. Procédé pour la préparation d'un mélange optiquement actif d'épimères en C(3) selon la revendication 1, caractérisé en ce qu'on réduit, à l'aide d'un aluminohydrure de métal alcalin, une cétone sous forme de l'un des énantiomères optiquement actifs de formule

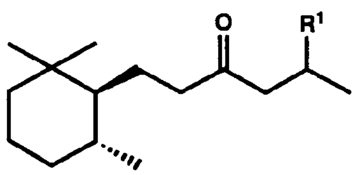

ou

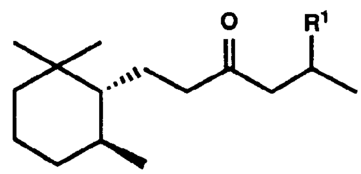

(-)-(1'S,6'R)
(IIIa)

(+)-(1'R,6'S)
(IIIb)

dans laquelle le symbole $R^1$ représente un atome d'hydrogène ou un radical méthyle, ou, respectivement, de formule

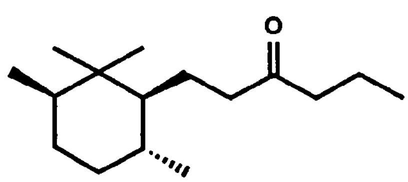

ou

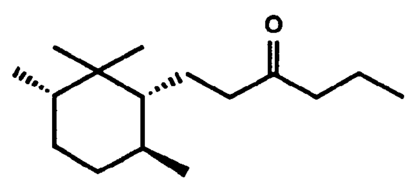

(-)-(1'S,3'R,6'R)
(IIIc)

(+)-(1'R,3'S,6'S)
(IIId)

pour obtenir un mélange d'épimères de formule (Ia) ou (Ib) ou, respectivement, un mélange d'épimères de formule (Ic) ou (Id).

4. Procédé pour la préparation d'un épimère en C(3) composant d'un mélange selon la revendication 1, caractérisé en ce qu'on fait réagir une hydroxy-sulfone de formule

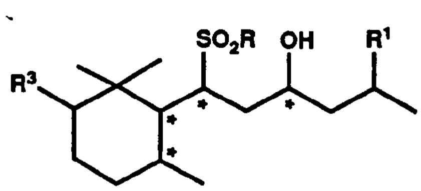

(IV)

dans laquelle l'astérisque indique la position d'un centre chiral, le symbole R représente un radical alkyle ou aryle et les symboles $R^1$ et $R^3$ sont définis comme à la revendication 1, sous forme d'un isomère optiquement actif approprié, avec un agent de clivage réducteur du groupe sulfonyle et on sépare l'épimère désiré du produit de la réaction.

5. Procédé selon la revendication 4, caractérisé en ce qu'on fait réagir un isomère optiquement actif approprié d'une hydroxy-sulfone de formule (IV), dans laquelle le symbole R représente un radical phényle, avec du naphtalure de lithium dans le tétrahydrofurane.

6. Utilisation d'un composé selon la revendication 1, à titre d'ingrédient parfumant, pour la préparation de compositions parfumantes et articles parfumés.

7. Utilisation selon la revendication 6 de (+)-(1'R,3S,6'S)-1-(2',2',6'-triméthyl-1'-cyclohexyl)-3-hexanol.

8. Utilisation selon la revendication 6 de (+)-(1'R,3'S,6'S)-1-(2',2',3',6'-tétraméthyl-1'-cyclohexyl)-3-hexanol.

9. Composition parfumante ou article parfumé résultant de l'utilisation selon l'une des revendications 6 à 8.

10. A titre d'article parfumé selon la revendication 9, un parfum ou une eau de toilette, un savon, un gel de douche ou bain, un shampoing ou un après-shampoing, un désodorisant corporel, une préparation cosmétique, un détergent ou revitalisant textile, un désodorisant d'air ambiant ou un produit d'entretien.

## Patentansprüche

1. Verbindung der Formel

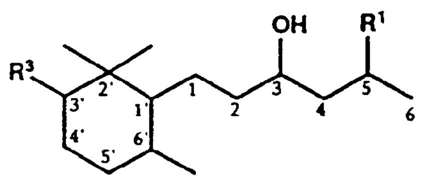

(I)

a) in welcher $R^3$ ein Wasserstoffatom bedeutet und $R^1$ ein Wasserstoffatom oder einen Methylrest bedeutet, in Form einer optisch aktiven Mischung von Epimeren bezüglich C(3) der Formel

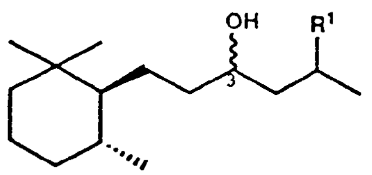

(-)-(1'S,6'R)
(Ia)

oder

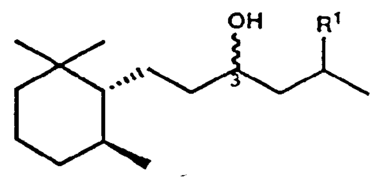

(+)-(1'R,6'S)
(Ib)

in welcher die Wellenlinie eine der beiden möglichen Orientierungen der OH-Gruppe anzeigt, oder in Form eines der diese Mischung bildenden Epimeren bezüglich C(3), oder
b) in welcher $R^3$ einen Methylrest bedeutet und $R^1$ ein Wasserstoffatom bedeutet, in Form einer optisch aktiven Mischung der Epimeren bezüglich C(3) der Formel

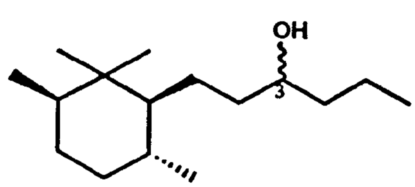

(-)-(1'S,3'R,6'R)
(Ic)

oder

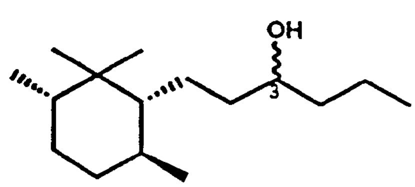

(+)-(1'R,3'S,6'S)
(Id)

in welcher die Wellenlinie die oben angegebene Bedeutung hat, oder in Form eines der die Mischung bildenden Epimeren bezüglich C(3).

2. Als Verbindung nach Anspruch 1 eines der optisch aktiven Diastereomeren der Formel

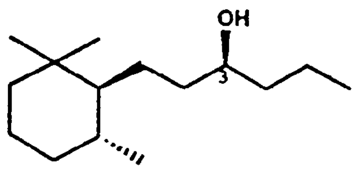

(-)-(1'S,3S,6'R)
(IIa)

oder

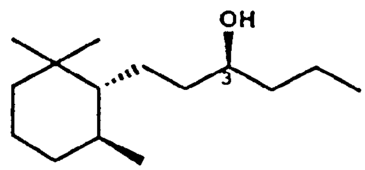

(+)-(1'R,3S,6'S)
(IIb)

oder sein Epimer bezüglich C(3).

3. Verfahren zur Herstellung einer optisch aktiven Mischung von Epimeren bezüglich C(3) nach Anspruch 1, dadurch gekennzeichnet, daß mit Hilfe eines Alkalimetallaluminiumhydrids ein Keton in Form eines der optisch aktiven Enantiomeren der Formel

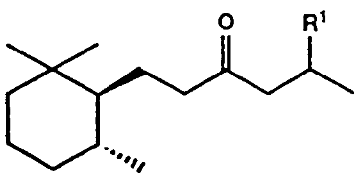

(-)-(1'S,6'R)
(IIIa)

oder

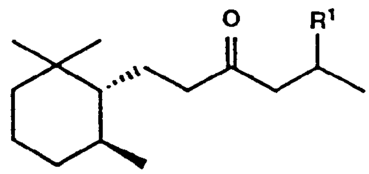

(+)-(1'R,6'S)
(IIIb)

in welcher $R^1$ ein Wasserstoffatom oder einen Methylrest bedeutet bzw. der Formel

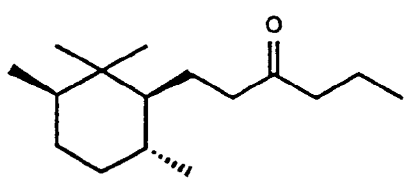

(-)-(1'S,3'R,6'R)
(IIIc)

oder

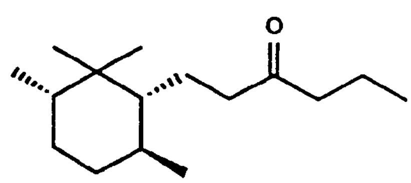

(+)-(1'R,3'S,6'S)
(IIId)

unter Bildung einer Mischung von Epimeren der Formel (Ia) oder (Ib) bzw. einer Mischung von Epimeren der Formel (Ic) oder (Id), reduziert wird.

4. Verfahren zur Herstellung eines Epimers bezüglich C(3) als Komponente einer Mischung nach Anspruch 1, dadurch gekennzeichnet, daß man eine Hydroxy-Sulfonverbindung der Formel

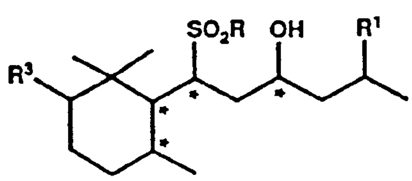

(IV)

in welchem das Sternchen die Stellung eines chiralen Zentrums anzeigt, R einen Alkyl- oder Arylrest bedeutet und $R^1$ und $R^3$ wie in Anspruch 1 definiert sind, in Form eines entsprechenden optisch aktiven

Isomers mit einem reduzierenden Abspaltungsmittel der Sulfonylgruppe umsetzt und das gewünschte Epimer des Reaktionsproduktes abtrennt.

5. Verfahren nach Anspruch 4, dadurch gekennzeichnet, daß man ein geeignetes optisch aktives Isomer einer Hydroxy-Sulfonverbindung der Formel (IV), in welcher R einen Phenylrest bedeutet, mit Lithiumnaphthalid in Tetrahydrofuran umsetzt.

6. Verwendung einer Verbindung nach Anspruch 1 als Riechstoffbestandteil zur Herstellung von Riechstoffkompositionen und parfümierter Artikel.

7. Verwendung nach Anspruch 6 von (+)-(1′R,3S,6′S)-1-(2′,2′,6′-Trimethyl-1′-cyclohexyl)-3-hexanol.

8. Verwendung nach Anspruch 6 von (+)-(1′R,3′S,6′S)-1-(2′,2′,3′,6′-Tetramethyl-1′-cyclohexyl)-3-hexanol.

9. Riechstoffkomposition oder parfümierter Artikel, erhalten durch die Verwendung nach einem der Ansprüche 6 bis 8.

10. Als parfümierter Artikel nach Anspruch 9 ein Parfüm oder Toilettwasser, eine Seife, ein Dusch- oder Badegel, Schampon oder Haarspülmittel, Körperdesodorierungsmittel, Kosmetikpräparat, Reinigungsmittel oder Textilauffrischungsmittel, Raumluftverbesserer oder Pflegeprodukt.

## Claims

1. Compound of formula

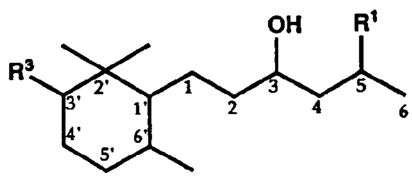

(I)

a) wherein symbol $R^3$ represents a hydrogen atom and symbol $R^1$ stands for a hydrogen atom or a methyl radical, in the form of an optically active mixture of C(3) epimers of formula

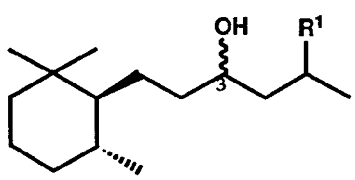

or

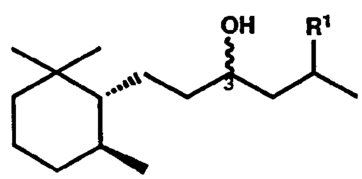

(-)-(1'S,6'R)
(Ia)

(+)-(1'R,6'S)
(Ib)

wherein the wavy line indicates one or the other of the possible orientations for the OH group, or in the form of one of the C(3) epimer constituents of said mixture; or
b) wherein symbol $R^3$ represents a methyl radical and symbol $R^1$ represents a hydrogen atom, in the form of an optically active mixture of C(3) epimers of formula

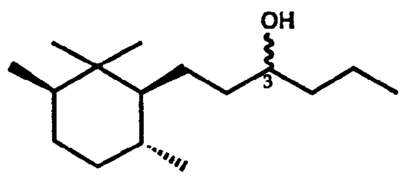 or 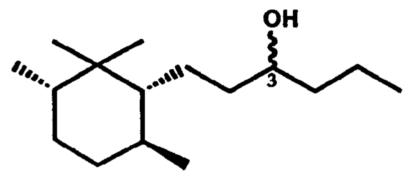

(-)-(1'S,3'R,6'R)
(Ic)

(+)-(1'R,3'S,6'S)
(Id)

wherein the wavy line has the meaning indicated above, or in the form of one of the C(3) epimer constituents of said mixture.

2. As the compound according to claim 1, an optically active diastereomer of formula

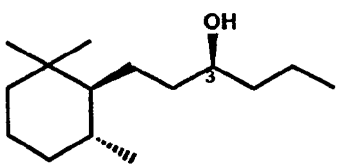 or 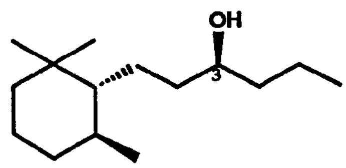

(-)-(1'S,3S,6'R)
(IIa)

(+)-(1'R,3S,6'S)
(IIb)

or its C(3) epimer.

3. Process for the preparation of an optically active mixture of C(3) epimers according to claim 1, characterized in that one reduces a ketone in the form of an optically active enantiomer of formula

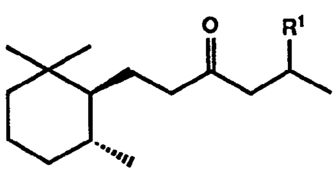 or 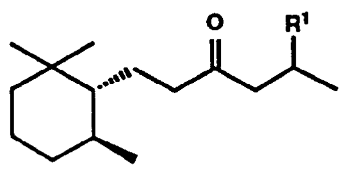

(-)-(1'S,6'R)
(IIIa)

(+)-(1'R,6'S)
(IIIb)

wherein symbol $R^1$ stands for a hydrogen atom or a methyl radical, or, respectively, of formula

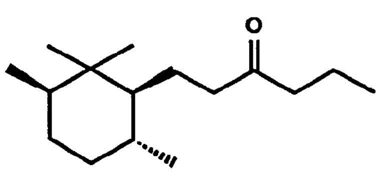 or 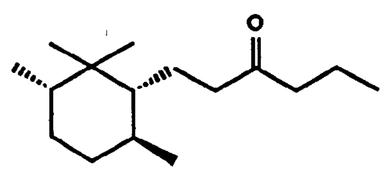

(-)-(1'S,3'R,6'R)
(IIIc)

(+)-(1'R,3'S,6'S)
(IIId)

by means of an alkali metal aluminohydride, to obtain a mixture of epimers of formula (Ia) or (Ib) or, respectively, a mixture of epimers of formula (Ic) or (Id).

4. Process for the preparation of a C(3) epimer component of a mixture according to claim 1, characterized in that one reacts a hydroxysulfone of formula

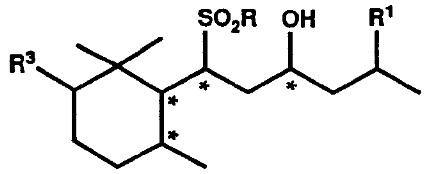

(IV)

wherein the asterisk indicates the location of a chiral center, symbol R stands for an alkyl or aryl radical and symbols $R^1$ and $R^3$ are defined as in claim 1, in the form of an appropriate optically active isomer, with an agent capable of the reductive cleavage of the sulfonyl group and in that the desired epimer is separated from the reaction product.

5. Process according to claim 4, characterized in that an appropriate optically active isomer of a hydroxysulfone of formula (IV), wherein symbol R stands for a phenyl radical, is reacted with lithium naphthalide in tetrahydrofuran.

6. Use of a compound according to claim 1 as a perfuming ingredient for the preparation of perfuming compositions and perfumed articles.

7. Use according to claim 6 of (+)-(1'R,3S,6'S)-1-(2',2',6'-trimethyl-1'-cyclohexyl)-3-hexanol.

8. Use according to claim 6 of (+)-(1'R,3'S,6'S)-1-(2',2',3',6'-tetramethyl-1'-cyclohexyl)-3-hexanol.

9. A perfuming composition or a perfumed article resulting from the use according to any one of claims 6 to 8.

10. As a perfumed article according to claim 9, a perfume or Cologne, a soap, a bath or shower gel, a shampoo or hair conditioner, a body deodorant, a cosmetic preparation, a detergent or fabric softener, an air freshener or a household product.